# DEMANDE DE BREVET EUROPEEN

(11) **EP 4 043 576 A1**
(43) Date de publication de la demande: **17.08.2022**
(21) Numéro de dépôt: 22151212.2
(22) Date de dépôt: 12.01.2022
(51) Int. Cl.: C12P 5/02, C12P 1/00, C12N 1/12, C12M 1/107, C12M 1/00, B01D 53/00, C01B 32/55

(54) **PROCÉDÉ ET INSTALLATION POUR LA PRODUCTION D'HYDROGÈNE GAZEUX ET DE CARBONE SOLIDE AVEC CONSOMMATION DE CO2**

(30) Priorité: 16.02.2021 FR 2101470
(71) Demandeur: EnerKa Conseil, 38950 Saint-Martin-le-Vinoux (FR)
(72) Inventeur: HUBERT, Karel, 38120 SAINT-EGREVE (FR)
(74) Mandataire: IP Trust

(57) **Abrégé**

Un procédé de production d'hydrogène gazeux et de carbone solide consistant à capter et valoriser gaz et fumées d'origine industrielle ou en sortie d'un réseau de chauffage directement sur le site libérant gaz et fumées avant leur rejet dans l'atmosphère. Le procédé comprend notamment les étapes de culture de biomasse à croissance rapide permettant une production de biométhane, ce dernier en mélange avec du méthane issu du réseau de distribution de gaz naturel, subit une décomposition thermique pour la production d'hydrogène gazeux et de carbone solide.

## Description

### Domaine technique

La présente invention concerne un procédé de production d'hydrogène gazeux et de carbone solide consistant à capter et valoriser gaz et fumées d'origine industrielle ou en sortie d'un réseau de chauffage directement sur le site libérant gaz et fumées avant leur rejet dans l'atmosphère. L'invention concerne également une installation pour la mise en œuvre du procédé.

### Technique antérieure

Dans un contexte de lutte contre le réchauffement climatique de nombreuses recherches visent à développer des procédés de captage et de valorisation de gaz et fumées d'origine industrielle riches en dioxyde de carbone avant leur rejet dans l'atmosphère.

Le document FR3049956 décrit un procédé de culture de microalgues à partir d'effluent de gaz industriel, afin de produire des molécules d'intérêts ou des biocarburants. Un inconvénient de ce procédé est que la biomasse produite doit être stockée et manutentionnée avant d'être valorisée.

Les documents GB1263231 et EP0890388 décrivent la production d'hydrogène gazeux et/ou de carbone solide à partir de dioxyde de carbone issu de l'atmosphère ou d'un gaz d'échappement. Le système de fixation de dioxyde de carbone de EP0890388 comprend en outre une cuve de fermentation de déchets organiques permettant la production de méthane gazeux qui par décomposition thermique conduit à la production d'hydrogène gazeux. Néanmoins, la production d'hydrogène gazeux à partir de ce procédé est énergivore et ne permet pas d'obtenir une chaine de valeur économiquement équilibrée ou avantageuse.

### Exposé de l'invention

Le but de l'invention est de proposer un nouveau procédé de production d'hydrogène gazeux et de carbone solide présentant une meilleure plus value économique et environnementale.

A cet effet, l'invention a pour objet un procédé de production d'hydrogène gazeux et de carbone solide consistant à capter et valoriser gaz et fumées d'origine industrielle ou en sortie d'un réseau de chauffage directement sur le site libérant gaz et fumées avant leur rejet dans l'atmosphère, le procédé comprenant les étapes suivantes :
a) la récupération de chaleur et l'extraction de dioxyde de carbone des gaz et fumées captés,
b) la fourniture du dioxyde de carbone extrait et de la chaleur récupérée à l'étape a) dans une cuve de bioréacteur renfermant un milieu nutritif à enrichir en dioxyde de carbone pour une culture de biomasse à croissance rapide composée de souche de microalgues, ou de macroalgues,
c) la transformation par fermentation anaérobie ou par gazéification hydrothermale de la biomasse cultivée à l'étape b) pour la production d'un digestat valorisable et d'un biogaz comprenant au moins du méthane et du dioxyde de carbone,
d) la séparation du digestat et du biogaz obtenus à l'étape c),
e) la séparation d'au moins du méthane et du dioxyde de carbone du biogaz produit à l'étape c),
f) la décomposition thermique du méthane pour produire du carbone à l'état solide et de l'hydrogène gazeux, caractérisé en ce que du méthane issu du réseau de distribution de gaz naturel peut être mélangé avec le méthane séparé à l'étape e) pour que le mélange de méthane subisse la décomposition thermique de l'étape f).

Lorsque à l'étape c) la transformation de la biomasse cultivée à l'étape b) est réalisée par gazéification hydrothermale pour la production d'un digestat valorisable et d'un biogaz, le biogaz produit comprend alors de l'hydrogène en plus du méthane et du dioxyde de carbone, et à l'étape e) l'hydrogène est séparé du méthane et du dioxyde de carbone du biogaz. Selon l'invention la production de méthane, appelé biométhane issu du biogaz produit à l'étape c) permet de consommer du CO₂ ce qui est bénéfique pour l'environnement.

Selon l'invention, il est avantageux de mélanger du méthane issu d'un réseau de gaz naturel avec du biométhane, pour avoir une chaine de valeur économique et environnementale équilibrée et positive.

Selon l'invention le procédé permet d'abaisser le coût de production de l'hydrogène gazeux ou « LCOH » (levelized cost of hydrogen).

Le procédé selon l'invention peut présenter les caractéristiques suivantes :
- la décomposition thermique du méthane à l'étape f) peut être réalisée par torche à plasma, ou par micro-ondes, ou par procédé thermique avec catalyseur métallique ou par procédé à haute température ;
- la chaleur récupérée des fumées captées peut être en outre utilisable à l'étape c) lors de la transformation par fermentation anaérobie de la biomasse ou la gazéification hydrothermale et/ou peut être utilisable à l'étape e) lors de la décomposition thermique du méthane ;
- le dioxyde de carbone séparé à l'étape e) du procédé peut être renvoyé vers le bioréacteur ;
- le digestat séparé à l'étape d) peut être renvoyé vers le bioréacteur.

Avantageusement, la biomasse produite est récoltée en vue de la valorisation du digestat dans les domaines phytosanitaire, agroalimentaire ou pharmaceutique. Avantageusement, des calories sont récupérables lors du refroidissement de fumées de sorte que de la chaleur fatale soit valorisable dans d'autres étapes du procédé, et le CO₂ extrait du biogaz soit ré-utilisable dans le procédé.

Une variante du procédé pourrait proposer que les étapes c) et suivantes puissent être réalisables sur un autre site que celui libérant gaz et fumées en insérant une étape de déplacement (par transport routier ou réseau d'assainissement et/ou collecte en station de traitement des eaux usées) de la biomasse cultivée entre l'étape b) et l'étape c).

La présente invention concerne également une installation de production de carbone solide et d'hydrogène gazeux conformément à la mise en œuvre du procédé selon l'invention, caractérisé en ce qu'elle peut comprendre :
- un moyen de prélèvement des gaz et fumées issus d'une source émettrice de gaz et fumées,
- un dispositif de culture de biomasse à croissance rapide composée de souche de microalgues, ou de macroalgues
- un dispositif de fermentation anaérobie pour la production de biogaz composé de méthane et de dioxyde de carbone par fermentation anaérobie de la culture de biomasse à croissance rapide ou d'un dispositif de gazéification hydrothermale pour la production de biogaz composé de méthane, de dioxyde de carbone et d'hydrogène par procédé thermochimique sur la culture de biomasse à croissance rapide,
- un séparateur de méthane du biogaz composé de méthane et de dioxyde de carbone généré par le dispositif de fermentation anaérobie ou du biogaz composé de méthane, de dioxyde de carbone et d'hydrogène généré par le dispositif de gazéification hydrothermale,
- un réacteur de décomposition de méthane pour la production d'hydrogène gazeux et de carbone solide à partir de méthane.

L'installation selon l'invention peut comprendre en outre au moins un échangeur de chaleur thermique pour récupérer de la chaleur des fumées industrielles ou celle générée par les moyens de formation d'hydrogène gazeux et de carbone solide pour la donner en outre au dispositif de culture et/ou au dispositif de fermentation anaérobie ou au dispositif de gazéification hydrothermale.

L'installation selon la présente invention peut ainsi être avantageusement utilisée pour le recyclage d'effluent gazeux et fumées d'origine industrielle ou en sortie d'un réseau de chauffage directement sur le site libérant gaz et fumées avant leur rejet dans l'atmosphère, mais aussi pour la production de d'hydrogène gazeux et de carbone solide à partir d'une culture de biomasse à croissance rapide.

### Description sommaire des dessins

La présente invention sera mieux comprise et d'autres caractéristiques et avantages apparaîtront à la lecture de la description détaillée des modes de réalisation pris à titre d'exemples nullement limitatifs et illustrée par le dessin annexé dans lequel :
[Fig 1] - la figure 1 est un diagramme illustrant une première variante du procédé et de l'installation selon l'invention ;
[Fig 2] - la figure 2 est un diagramme illustrant une seconde variante du procédé et de l'installation selon l'invention.

### Description des modes de réalisation

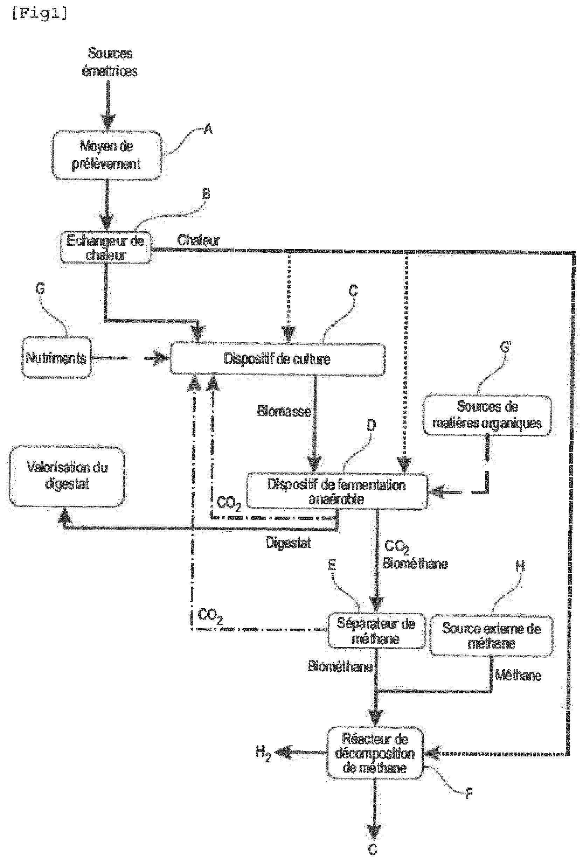

Le diagramme de la figure 1 illustre une première variante du procédé et de l'installation pour la mise en œuvre du procédé de la présente invention. Les flèches en trait en pointillés illustrent des voies optionnelles.

L'installation comprend un moyen de prélèvement (A) des gaz et fumées issus d'une ou de plusieurs sources émettrices, au moins un échangeur de chaleur (B), un dispositif de culture (C) de biomasse à croissance rapide, un dispositif de fermentation anaérobie (D), un séparateur (E) de méthane, un réacteur (F) de décomposition de méthane pour la production d'hydrogène gazeux et de carbone solide.

Selon l'invention, les gaz et fumées contenant en outre du CO₂, NOx, SOx, des particules fines et de la chaleur, venant de sources émettrices d'origine industrielle ou en sortie d'un réseau de chauffage, sont captés par le moyen de prélèvement (A) avant leur rejet dans l'atmosphère, par exemple dans un conduit d'une cheminée. Les gaz et fumées passent à travers l'échangeur de chaleur (B) de sorte à récupérer des calories extraites de la chaleur fatale libérée, calories qui peuvent être réutilisées dans l'installation. Après passage dans l'échangeur de chaleur (B), les gaz « refroidis » peuvent optionnellement être traités avant leur introduction dans une cuve de bioréacteur du dispositif de culture (C) pour enrichir notamment en dioxyde de carbone un milieu nutritif de culture. Une culture de biomasse à croissance rapide, telle une culture de biomasse composée de souche de microalgues, ou macroalgues, est réalisée dans la cuve de bioréacteur. Un apport externe de nutriments (G) est possible pour enrichir le milieu nutritif. De manière avantageuse, les calories récupérées par l'échangeur de chaleur (B) sont utilisables dans le dispositif de culture (C) pour optimiser la culture de biomasse.

La biomasse cultivée produite est ensuite prélevée puis transformée dans le dispositif de fermentation anaérobie (D) dans une cuve de fermentation selon un processus de type biologique connu de méthanisation. Optionnellement un apport d'autres sources de matières organiques (G'), par exemple d'origine industrielle ou agricole, peuvent être ajoutées à la biomasse cultivée dans la cuve de fermentation. De manière avantageuse, les calories récupérées par l'échangeur de chaleur (B) sont aussi utilisables dans le dispositif de fermentation anaérobie (D) pour optimiser la fermentation anaérobie.

En fin de cycle de fermentation anaérobie, un digestat valorisable et un biogaz contenant du méthane (appelé biométhane ou méthane renouvelable) et du dioxyde de carbone sont produits et récupérables. Ainsi, digestat et biogaz sont séparés de sorte que le digestat soit extrait pour être valorisé par exemple dans l'alimentation animale ou humaine.

Le biométhane du mélange gazeux méthane et dioxyde de carbone du biogaz produit est extrait par le séparateur de méthane (E) puis le méthane peut être concentré selon toutes méthodes connues.

Le dioxyde de carbone issu du biogaz peut être optionnellement introduit dans le dispositif de culture (C) pour enrichir le milieu nutritif de culture.

Le biométhane est orienté vers un réacteur de décomposition de méthane (F) de sorte à subir une étape de décomposition thermique, par exemple par les méthodes précitées, pour produire de l'hydrogène gazeux et du carbone solide selon la réaction CH₄ -> 2H₂ + C.

L'hydrogène gazeux ainsi produit peut être purifié, compressé, stocké ou transporté pour être conditionné et/ou vendu, ou être utilisé, par exemple, pour la production d'électricité par une pile à combustible.

Le carbone solide ainsi produit peut être valorisé dans l'industrie, par exemple le secteur des pneumatiques ou des encres.

Selon l'invention, avant que le biométhane produit subisse la réaction de décomposition thermique, celui-ci peut être mélangé avec une source externe de méthane (H), comme par exemple du méthane issu du réseau de gaz naturel afin d'augmenter la quantité de méthane qui subira l'étape de décomposition thermique. Un tel mélange biométhane / méthane est avantageux économiquement pour la production d'hydrogène gazeux et de carbone solide par le procédé et l'installation selon l'invention. Dans le secteur gazier, il est connu d'injecter dans le réseau de gaz naturel du biométhane produit mais il n'est pas connu, ni évident, de ponctionner du méthane dit « fossile » hors du réseau pour le mélanger à du biométhane produit, pour produire de l'hydrogène gazeux et du carbone solide tout en consommant du CO₂.

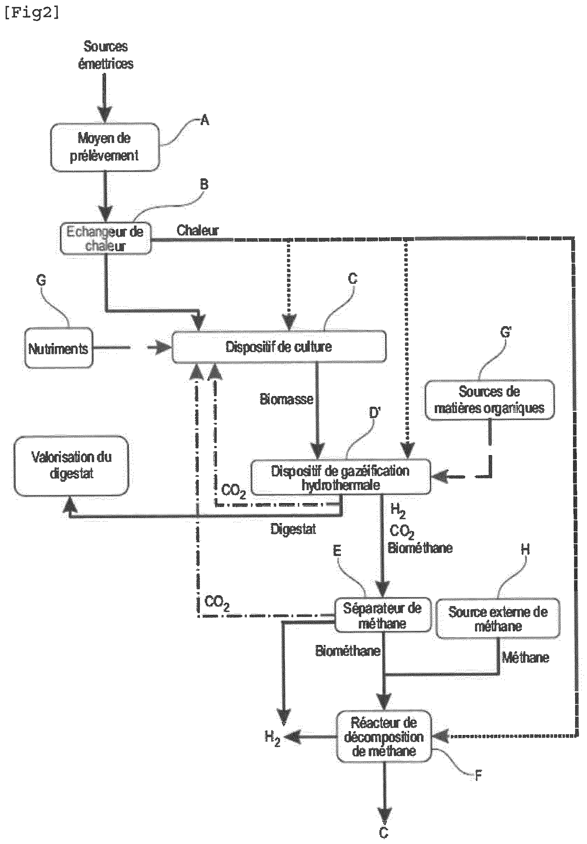

Selon une seconde variante de l'invention illustré sur le diagramme de la figure 2, la biomasse cultivée peut aussi être transformée dans un dispositif de gazéification hydrothermale (D') selon un processus de type thermochimique connu dans un gazéifieur, en remplacement du dispositif de fermentation anaérobie décrit précédemment. Dans le cas d'une transformation de la biomasse dans un dispositif de gazéification hydrothermale (D'), le biogaz produit comprendra de l'hydrogène, en plus du méthane et du dioxyde de carbone. L'hydrogène gazeux ainsi produit sera séparé de façon connue du méthane et du dioxyde de carbone du biogaz par le séparateur de méthane (E) puis l'hydrogène est traité comme décrit précédemment pour par exemple la production d'électricité par une pile à combustible. Le méthane isolé et le dioxyde de carbone isolé sont utilisés comme précédemment décrit.

Il va de soi que la présente invention ne saurait être limitée au mode de réalisation exposé plus haut, susceptible de subir des modifications sans pour autant sortir du cadre de l'invention.

## Revendications

1. Procédé de production d'hydrogène gazeux et de carbone solide consistant à capter et valoriser gaz et fumées d'origine industrielle ou en sortie d'un réseau de chauffage directement sur le site libérant gaz et fumées avant leur rejet dans l'atmosphère, ledit procédé comprenant les étapes suivantes :
a) la récupération de chaleur et l'extraction de dioxyde de carbone desdits gaz et fumées captés,
b) la fourniture dudit dioxyde de carbone extrait et de ladite chaleur récupérée à l'étape a) dans une cuve de bioréacteur renfermant un milieu nutritif à enrichir en dioxyde de carbone pour une culture de biomasse à croissance rapide composée de souche de microalgues, ou de macroalgues,
c) la transformation par fermentation anaérobie ou par gazéification hydrothermale de ladite biomasse cultivée à l'étape b) pour la production d'un digestat valorisable et d'un biogaz comprenant au moins du méthane et du dioxyde de carbone,
d) la séparation dudit digestat et dudit biogaz obtenus à l'étape c),
e) la séparation d'au moins dudit méthane et dudit dioxyde de carbone dudit biogaz produit à l'étape c),
f) la décomposition thermique du méthane pour produire du carbone à l'état solide et de l'hydrogène gazeux,
**caractérisé en ce que** du méthane issu du réseau de distribution de gaz naturel est mélangé avec ledit méthane séparé à l'étape e) pour que ledit mélange de méthane subisse ladite décomposition thermique de l'étape f).

2. Procédé selon la revendication 1, **caractérisé en ce que** à l'étape c) ledit biogaz produit lors de ladite transformation par gazéification hydrothermale de ladite biomasse cultivée à l'étape b) comprend du méthane, du dioxyde de carbone et de l'hydrogène, et **en ce que** à l'étape e) ledit hydrogène est séparé dudit méthane et dudit dioxyde de carbone dudit biogaz.

3. Procédé selon la revendication 1 ou 2, **caractérisé en ce que** ladite décomposition thermique du méthane à l'étape f) est réalisée par torche à plasma, ou par micro-ondes, ou par procédé thermique avec catalyseur métallique ou par procédé à haute température.

4. Procédé selon l'une des revendications précédentes, **caractérisé en ce que** ladite chaleur récupérée desdites fumées captées est en outre utilisable à l'étape c) lors de ladite transformation par fermentation anaérobie ou par gazéification hydrothermale de ladite biomasse et/ou est utilisable à l'étape e) lors de ladite décomposition thermique du méthane.

5. Procédé selon l'une des revendications précédentes, **caractérisé en ce que** ledit dioxyde de carbone séparé à l'étape e) est renvoyé vers ledit bioréacteur.

6. Procédé selon l'une des revendications précédentes, **caractérisé en ce que** ledit digestat séparé à l'étape d) est renvoyé vers ledit bioréacteur.

7. Installation pour la mise en œuvre du procédé selon l'une des revendications précédentes, **caractérisé en ce qu'**elle comprend :
- un moyen de prélèvement (A) des gaz et fumées issus d'une source émettrice de gaz et fumées,
- un dispositif de culture (C) de biomasse à croissance rapide composée de souche de microalgues, ou de macroalgues,
- un dispositif de fermentation anaérobie (D) pour la production de biogaz composé de méthane et de dioxyde de carbone par fermentation anaérobie de la culture de biomasse à croissance rapide ou d'un dispositif de gazéification hydrothermale (D') pour la production de biogaz composé de méthane, de dioxyde de carbone et d'hydrogène par procédé thermochimique sur la culture de biomasse à croissance rapide,
- un séparateur de méthane (E) du biogaz composé de méthane et de dioxyde de carbone généré par ledit dispositif de fermentation anaérobie (D) ou du biogaz composé de méthane, de dioxyde de carbone et d'hydrogène généré par ledit dispositif de gazéification hydrothermale (D'),
- un réacteur de décomposition de méthane (F) pour la production d'hydrogène et de carbone solide à partir de méthane.

8. Installation selon la revendication 7, **caractérisé en ce qu'**il comprend en outre au moins un échangeur de chaleur (B) thermique pour récupérer de la chaleur des fumées ou celle générée par des moyens de formation d'hydrogène gazeux et de carbone solide pour la donner en outre audit dispositif de culture (C) de biomasse et/ou audit dispositif de fermentation anaérobie (D) ou audit dispositif de gazéification hydrothermale (D').
